# EUROPÄISCHE PATENTSCHRIFT

(11) **EP 0 828 734 B1**
(45) Veröffentlichungstag und Bekanntmachung des Hinweises auf die Patenterteilung: **23.08.2000**
(21) Anmeldenummer: 96919790.4
(22) Anmeldetag: 20.05.1996
(51) Int. Cl.: C07D 405/06, A01N 43/653

(54) **TRIAZOLYLMETHYL-OXIRANE**
TRIAZOLYL METHYL OXIRANES
TRIAZOLYLMETHYL-OXIRANNES

(30) Priorität: 01.06.1995 DE 19520097
(43) Veröffentlichungstag der Anmeldung: 18.03.1998
(73) Patentinhaber: BAYER AG, 51368 Leverkusen (DE)
(72) Erfinder: JAUTELAT, Manfred, D-51399 Burscheid (DE); TIEMANN, Ralf, D-51375 Leverkusen (DE); DUTZMANN, Stefan, D-40721 Hilden (DE); STENZEL, Klaus, D-40595 Düsseldorf (DE)
(86) Internationale Anmeldenummer: EP9602165
(87) Internationale Veröffentlichungsnummer: WO9638440

(56) Entgegenhaltungen:
- EP-A- 0 094 564
- EP-A- 0 196 038
- EP-A- 0 327 913
- EP-A- 0 352 675

## Beschreibung

Die vorliegende Erfindung betrifft neue Triazolylmethyl-oxirane, ein Verfahren zu deren Herstellung und deren Verwendung als Mikrobizide.

Es ist bereits bekannt geworden, daß zahlreiche Azolylmethyl-oxiran-Derivate fungizide Eigenschaften besitzen (vgl. EP-A 0 094 564 und EP-A 0 196 038). So läßt sich z.B. 3-(2-Chlor-phenyl)-2-(4-fluor-phenyl)-2-(1,2,4-triazol-1-yl-methyl)-oxiran zur Bekämpfung von Pilzen verwenden. Die Wirksamkeit dieses Stoffes ist gut, läßt aber bei niedrigen Aufwandmengen in manchen Fällen zu wünschen übrig.

Es wurden nun neue Triazolylmethyl-oxirane der Formel 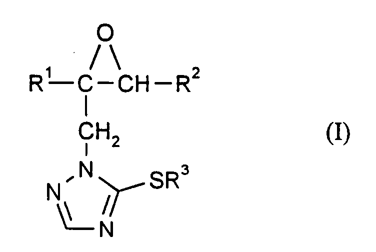 in welcher
- R¹: für Alkyl mit 1 bis 4 Kohlenstoffatomen, Halogenalkyl mit 1 bis 4 Kohlenstoffatomen und 1 bis 5 Halogenatomen, gegebenenfalls durch Halogen substituiertes Cycloalkyl mit 3 bis 7 Kohlenstoffatomen, Naphthyl oder gegebenenfalls einfach bis dreifach, gleichartig oder verschieden durch Halogen, Nitro, Phenyl, Phenoxy, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen und/oder Halogenalkylthio mit 1 bis 4 Kohlenstoffatomen und 1 bis 5 Halogenatomen substituiertes Phenyl steht,
- R²: für gegebenenfalls einfach bis dreifach, gleichartig oder verschieden durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen und/oder Halogenalkylthio mit 1 bis 4 Kohlenstoffatomen und 1 bis 5 Halogenatomen substituiertes Phenyl steht und
- R³: für Wasserstoff oder Alkyl mit 1 bis 4 Kohlenstoffatomen steht,
sowie deren Säureadditions-Salze und Metallsalz-Komplexe gefunden.

Die erfindungsgemäßen Stoffe enthalten zwei asymmetrisch substituierte Kohlenstoffatome und können deshalb in Form von Diastereomeren oder Enantiomeren anfallen. Die vorliegende Erfindung betrifft sowohl die einzelnen Isomeren als auch deren Gemische.

Weiterhin wurde gefunden, daß man Triazolylmethyl-oxirane der Formel (I) sowie deren Säureadditions-Salze und Metallsalz-Komplexe erhält, wenn man Oxiran-Derivate der Formel 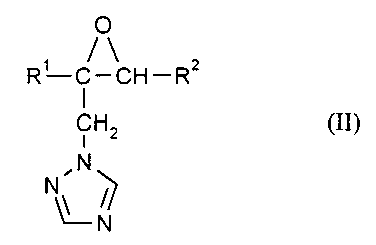 in welcher
R¹ und R² die oben angegebenen Bedeutungen haben,
nacheinander mit starken Basen und Schwefel in Gegenwart eines Verdünnungsmittels umsetzt und dann mit Wasser, gegebenenfalls in Gegenwart einer Säure hydrolysiert und gegebenenfalls die dabei entstehenden Verbindungen der Formel 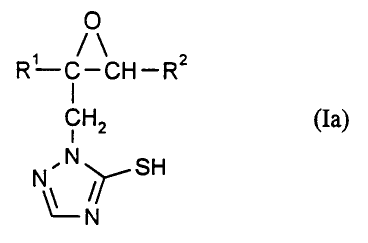 in welcher
R¹ und R² die oben angegebenen Bedeutungen haben,
mit Halogen-Verbindungen der Formel

R⁴-Hal (IV)

in welcher
- R⁴: für Alkyl mit 1 bis 4 Kohlenstoffatomen steht und
- Hal: für Chlor, Brom oder Iod steht,
in Gegenwart eines Säurebindemittels und in Gegenwart eines Verdünnungsmittels umsetzt,
und gegebenenfalls anschließend an die so erhaltenen Verbindungen der Formel (I) eine Säure oder ein Metallsalz addiert.

Schließlich wurde gefunden, daß die neuen Triazolylmethyl-oxirane der Formel (I) sowie deren Säureadditions-Salze und Metallsalz-Komplexe sehr gute mikrobizide Eigenschaften aufweisen und sowohl im Pflanzenschutz als auch im Materialschutz eingesetzt werden können.

Überraschenderweise besitzen die erfindungsgemäßen Stoffe eine bessere mikrobizide Wirksamkeit als die konstitutionell ähnlichsten, vorbekannten Verbindungen gleicher Wirkungsrichtung. So übertreffen die erfindungsgemäßen Stoffe das 3-(2-Chlorphenyl)-2-(4-fluor-phenyl)-2-(1,2,4-triazol-1-yl-methyl)-oxiran bezüglich der fungiziden Eigenschaften.

Die erfindungsgemäßen Triazolylmethyl-oxirane sind durch die Formel (I) allgemein definiert.
- R¹: steht vorzugsweise für Methyl, Ethyl, n-Propyl, Isopropyl, n-Butyl, sek.-Butyl, iso-Butyl, tert.-Butyl, Fluor-tert.-butyl, Difluor-tert.-butyl, gegebenenfalls einfach bis dreifach, gleichartig oder verschieden durch Fluor, Chlor und/oder Brom substituiertes Cycloalkyl mit 3 bis 6 Kohlenstoffatomen, für Naphthyl oder für Phenyl, das einfach bis dreifach, gleichartig oder verschieden substituiert sein kann durch Fluor, Chlor, Brom, Nitro, Phenyl, Phenoxy, Methyl, Ethyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio.
- R²: steht vorzugsweise für Phenyl, das einfach bis dreifach, gleichartig oder verschieden substituiert sein kann durch Fluor, Chlor, Brom, Methyl, Ethyl, Isopropyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio.
- R³: steht vorzugsweise für Wasserstoff, Methyl oder Ethyl.

Bevorzugte erfindungsgemäße Verbindungen sind auch Additionsprodukte aus Säuren und denjenigen Triazolylmethyl-oxiranen der Formel (I), in denen R¹, R² und R³ diejenigen Bedeutungen haben, die für diese Substituenten als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure oder Camphersulfonsäure, Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Triazolylmethyl-oxiranen der Formel (I), in denen R¹, R² und R³ diejenigen Bedeutungen haben, die für diese Substituenten als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Die erfindungsgemäßen Triazolylmethyl-oxirane der Formel (I), in denen R³ für Wasserstoff steht, können in der "Mercapto"-Form der Formel 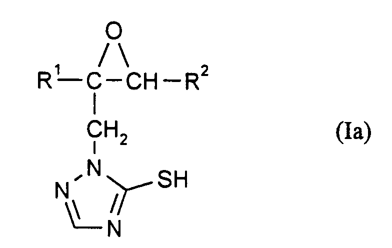 oder in der tautomeren "Thiono"-Form der Formel 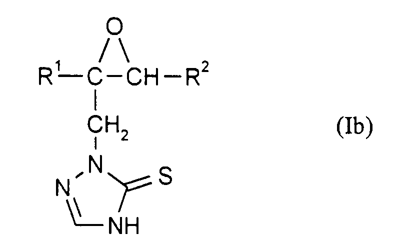 vorliegen. Der Einfachheit halber wird jeweils nur die "Mercapto"-Form aufgeführt.

Als Beispiele für erfindungsgemäße Stoffe seien die in der folgenden Tabelle aufgeführten Triazolylmethyl-oxirane genannt.

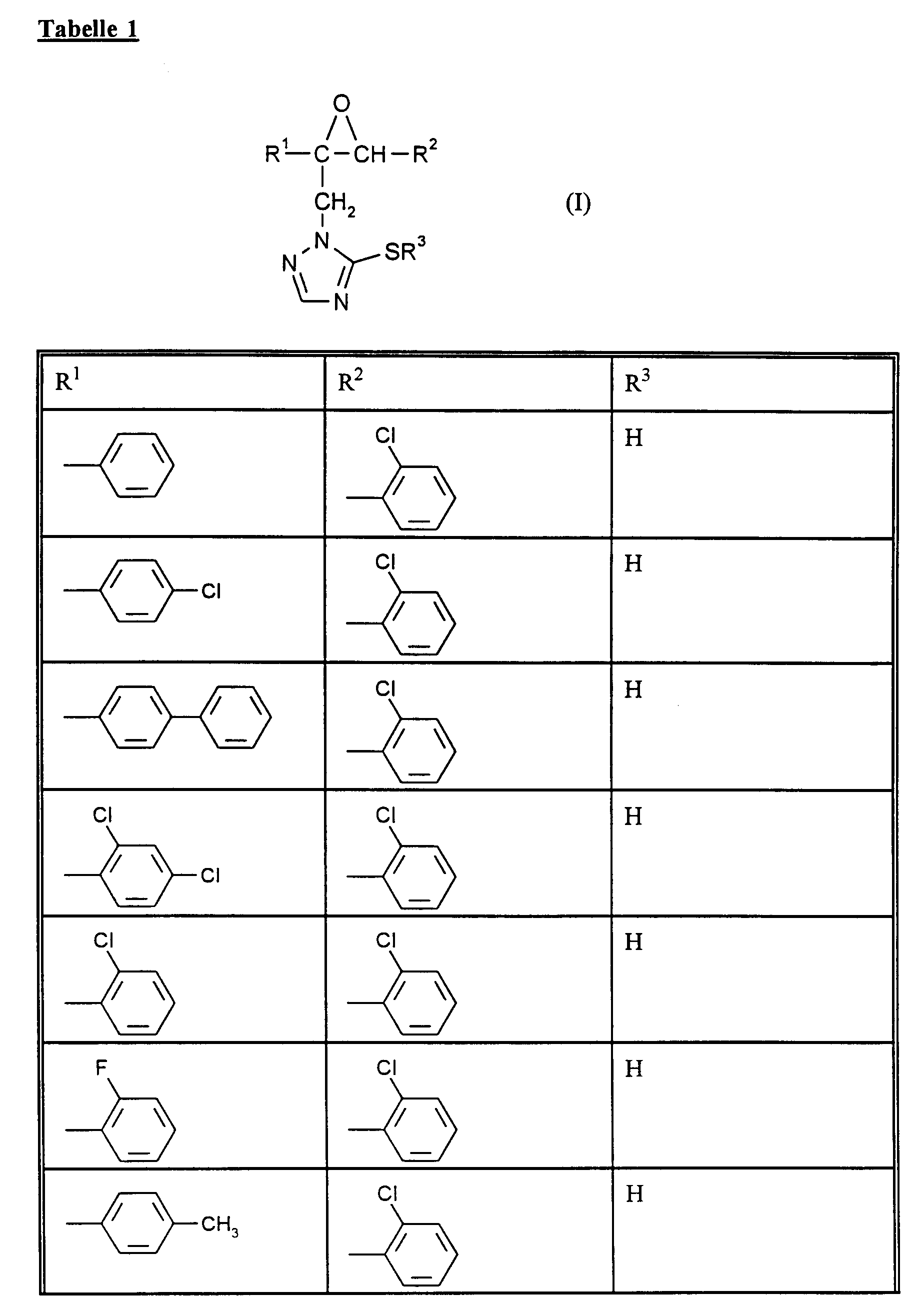

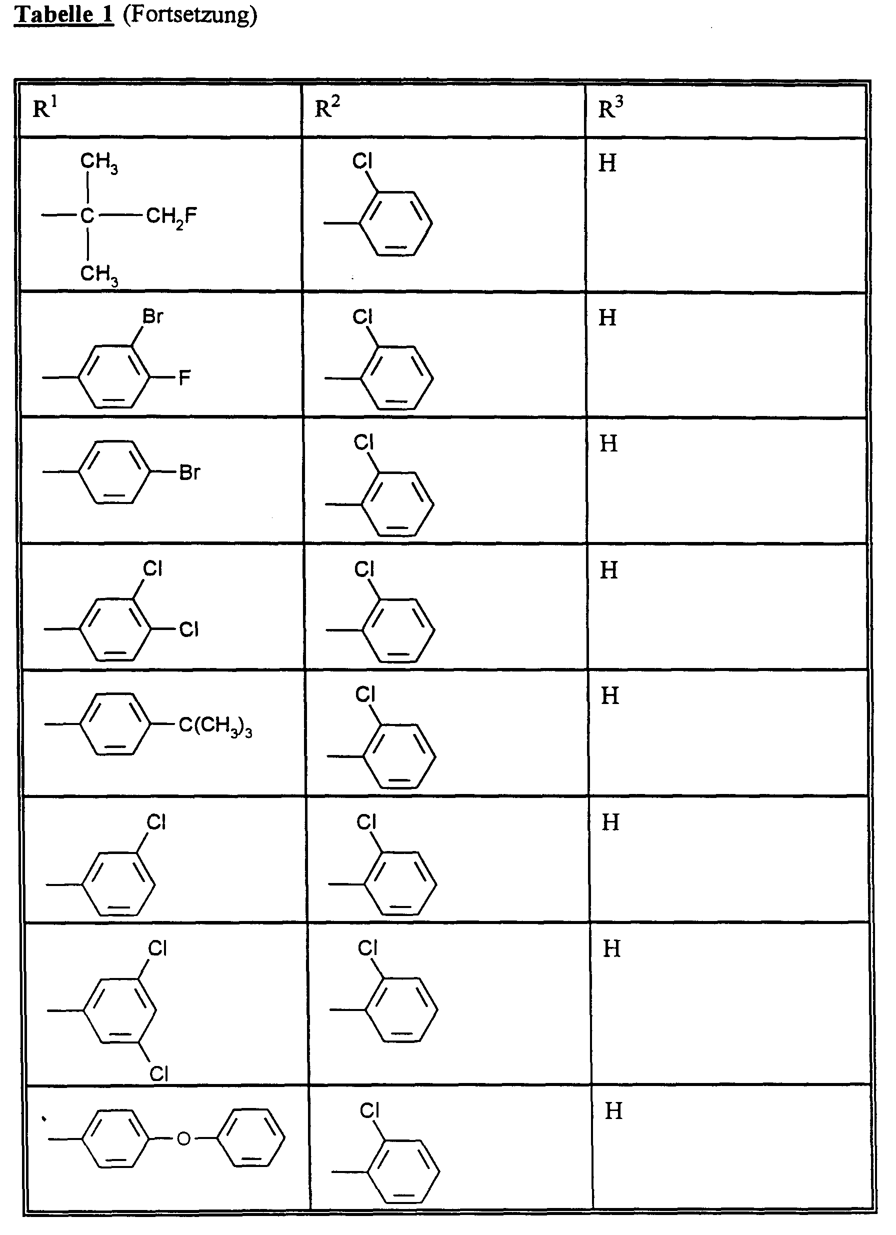

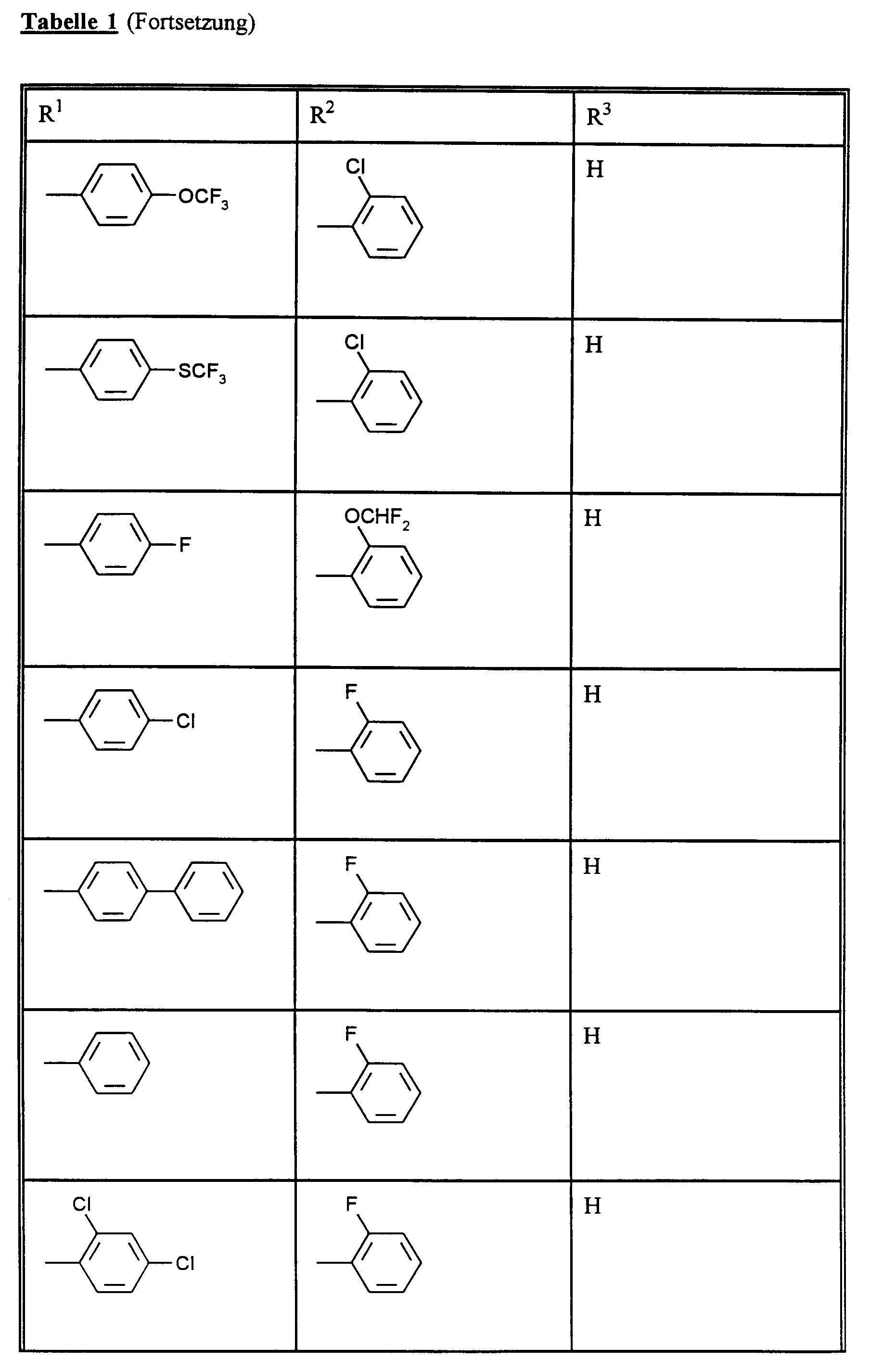

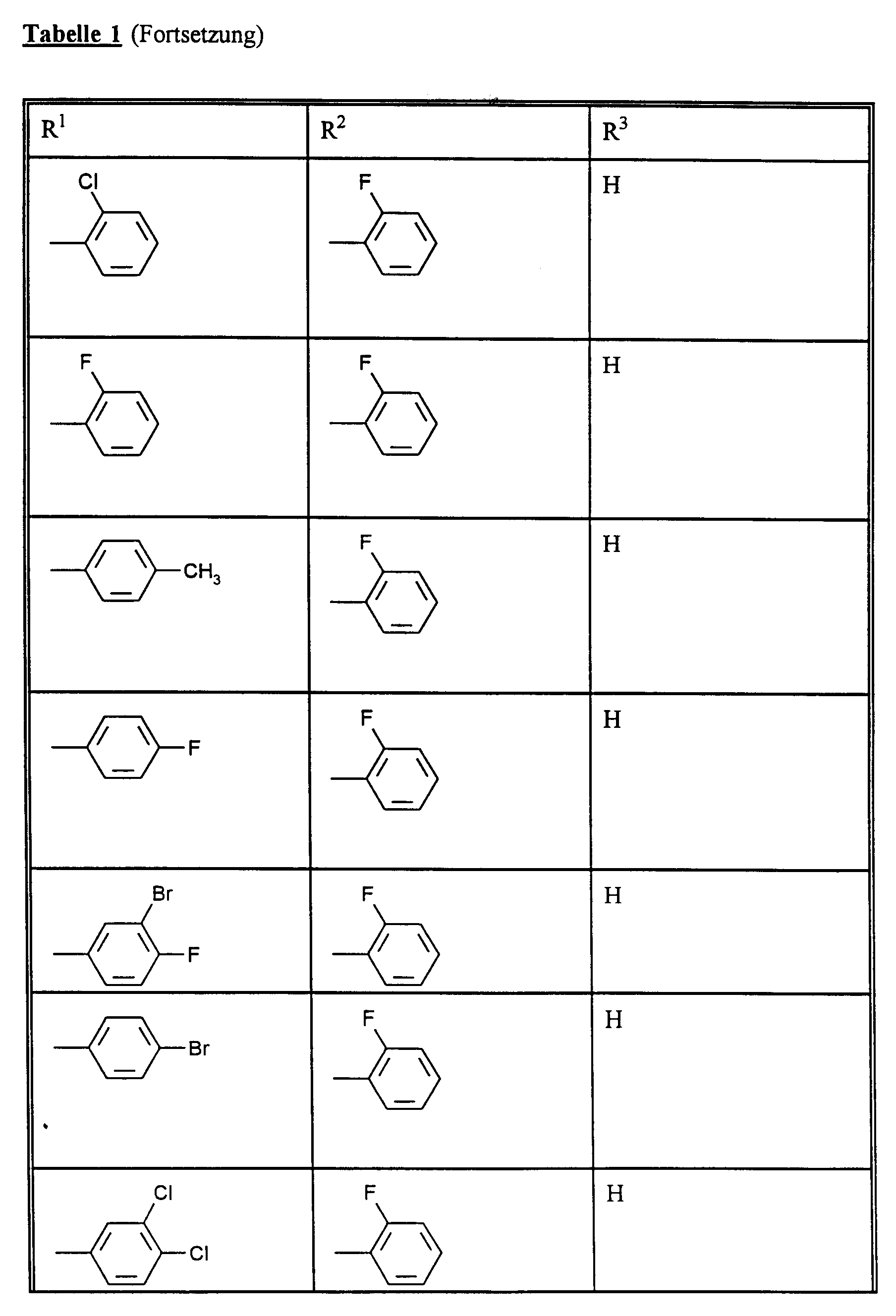

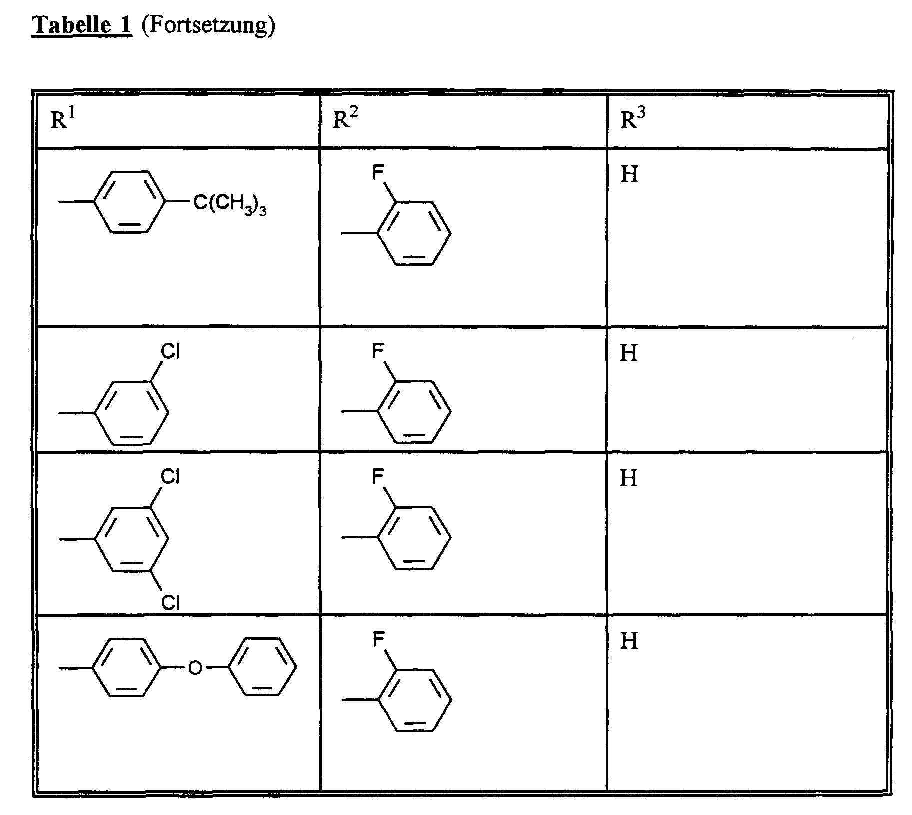

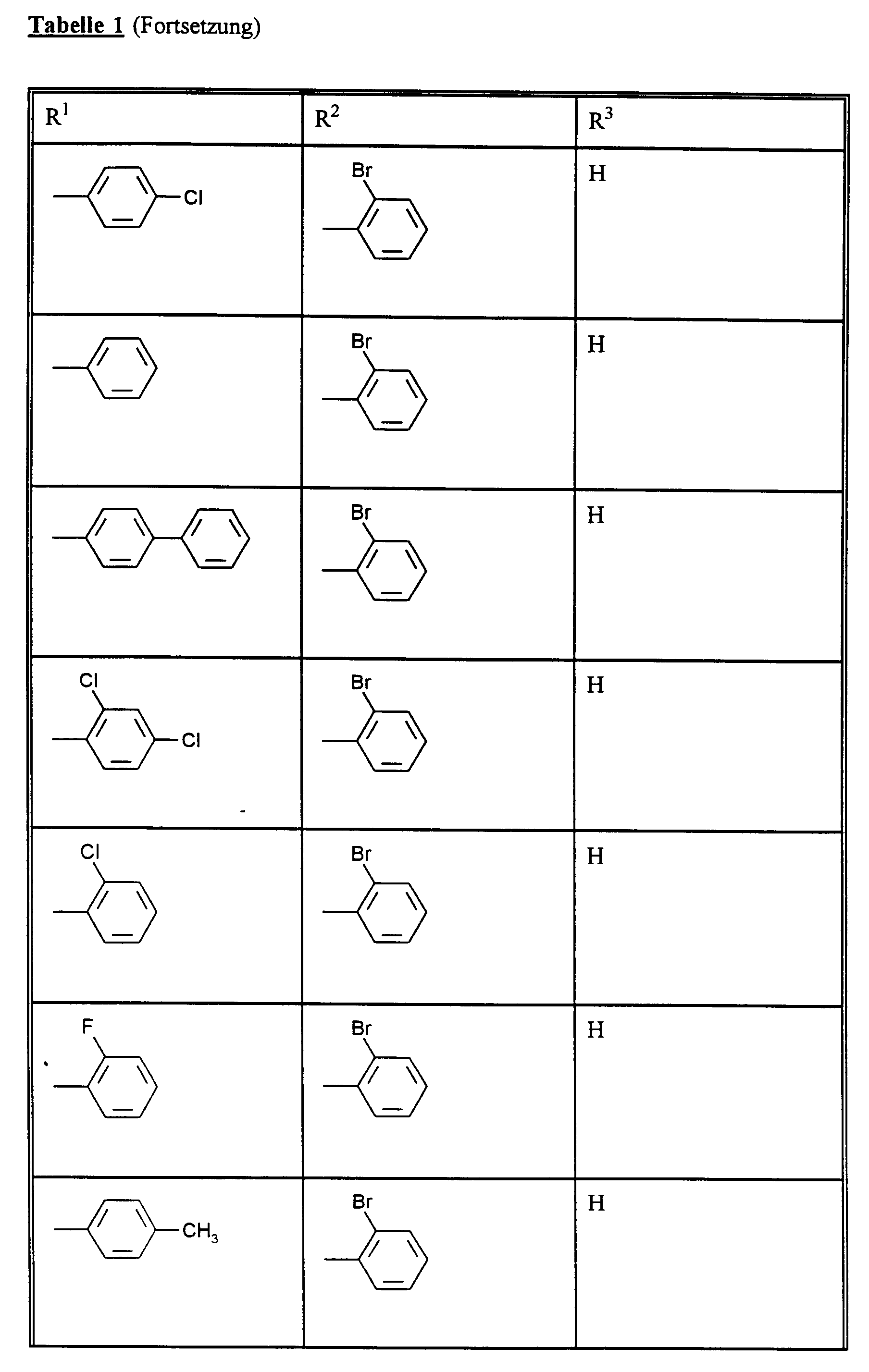

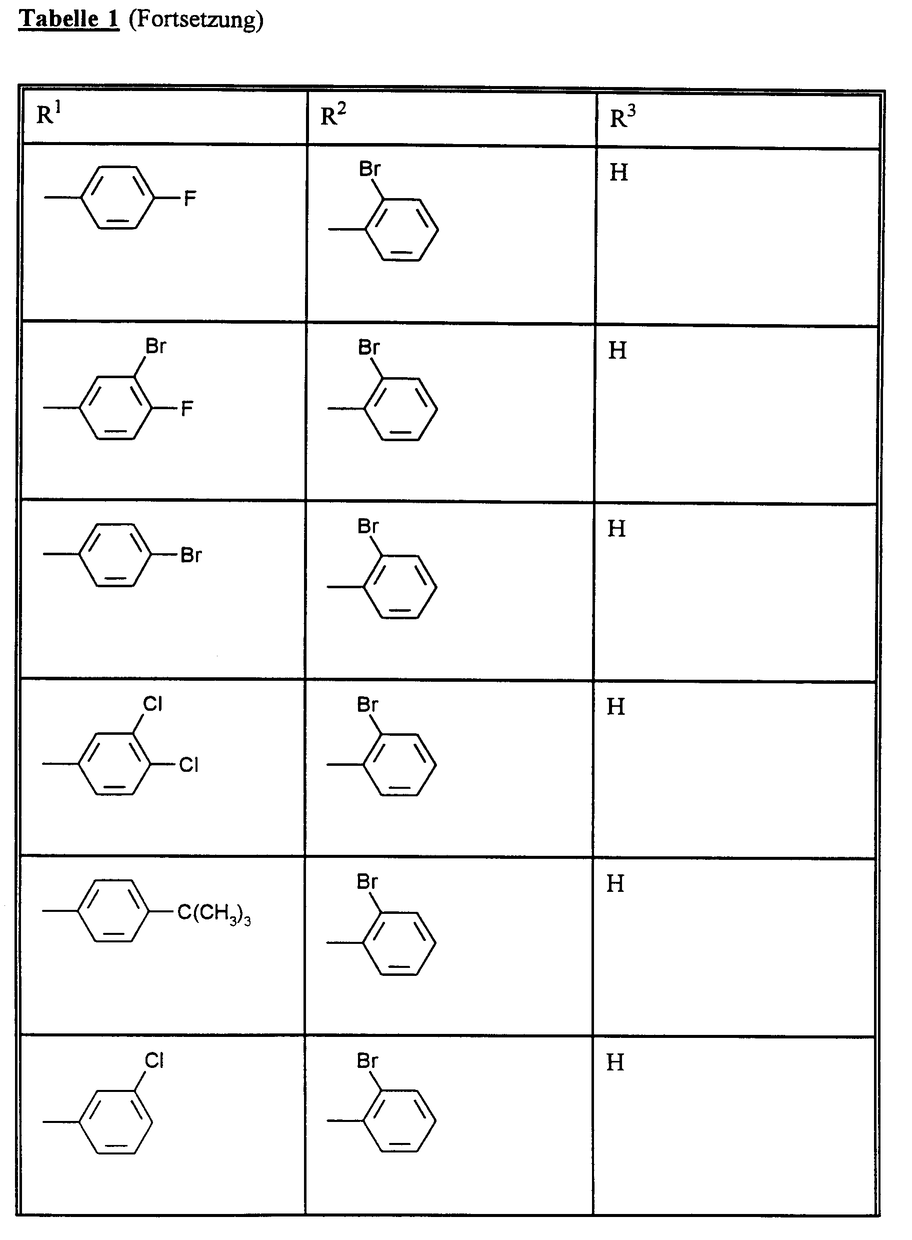

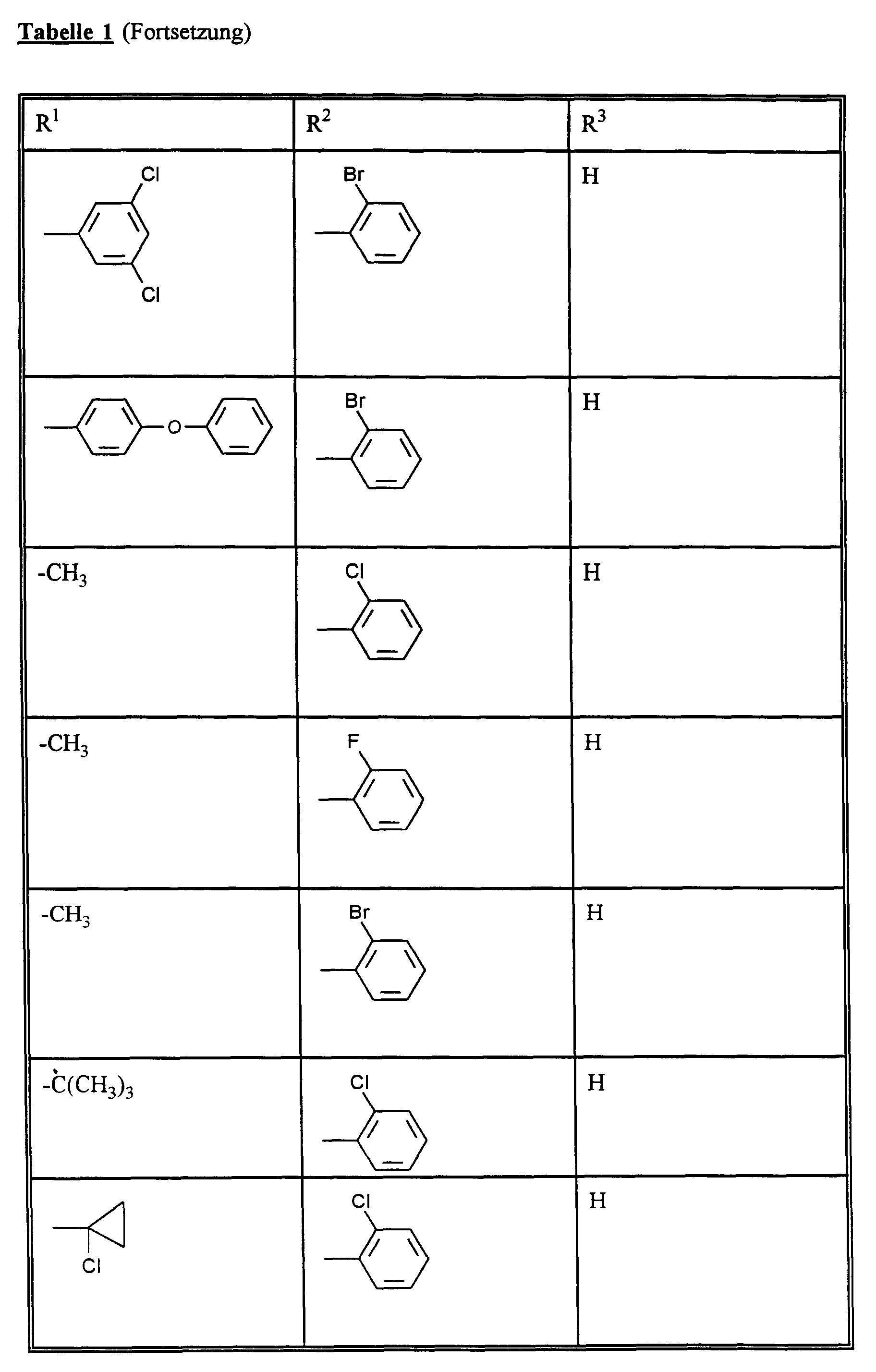

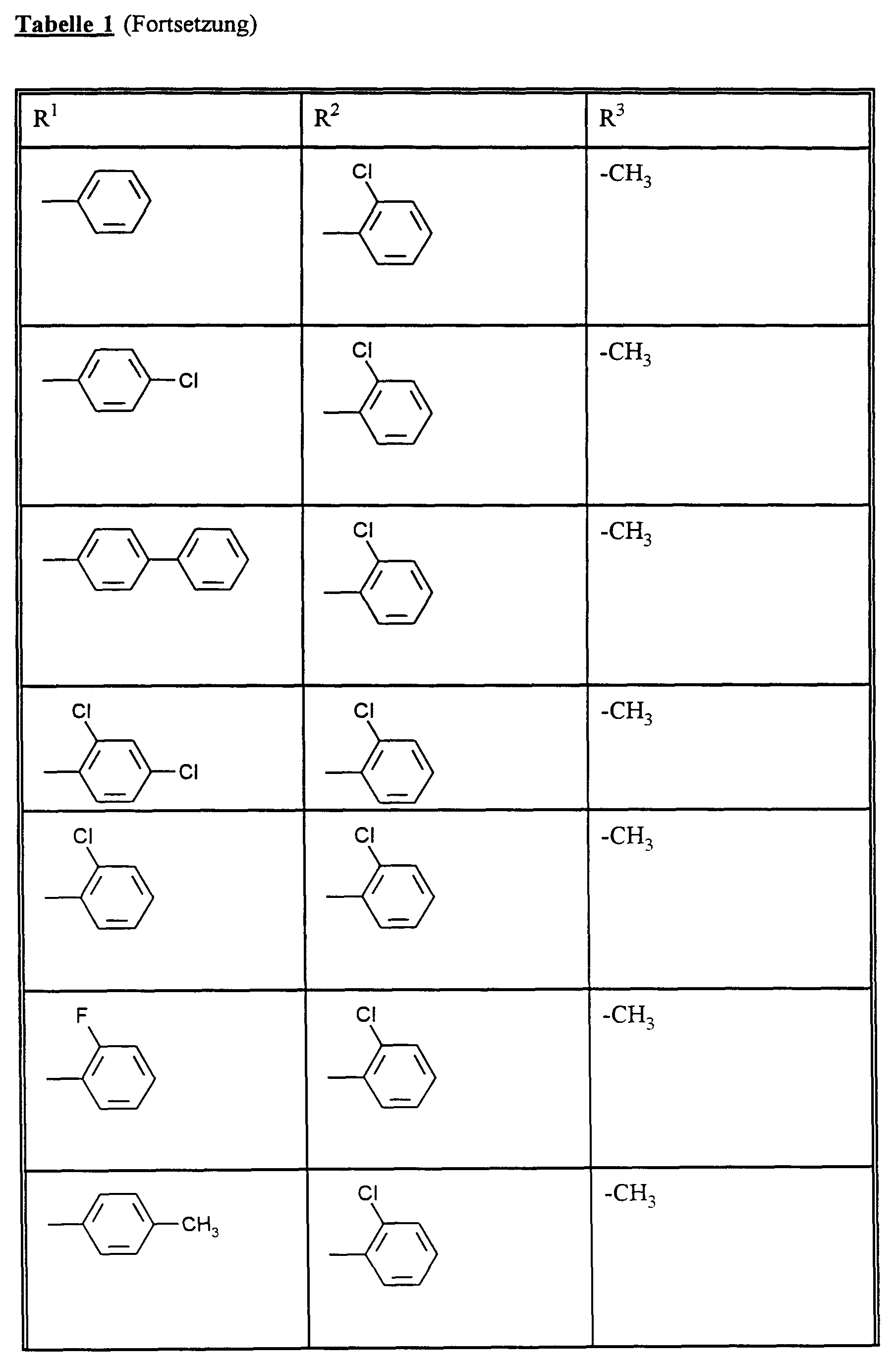

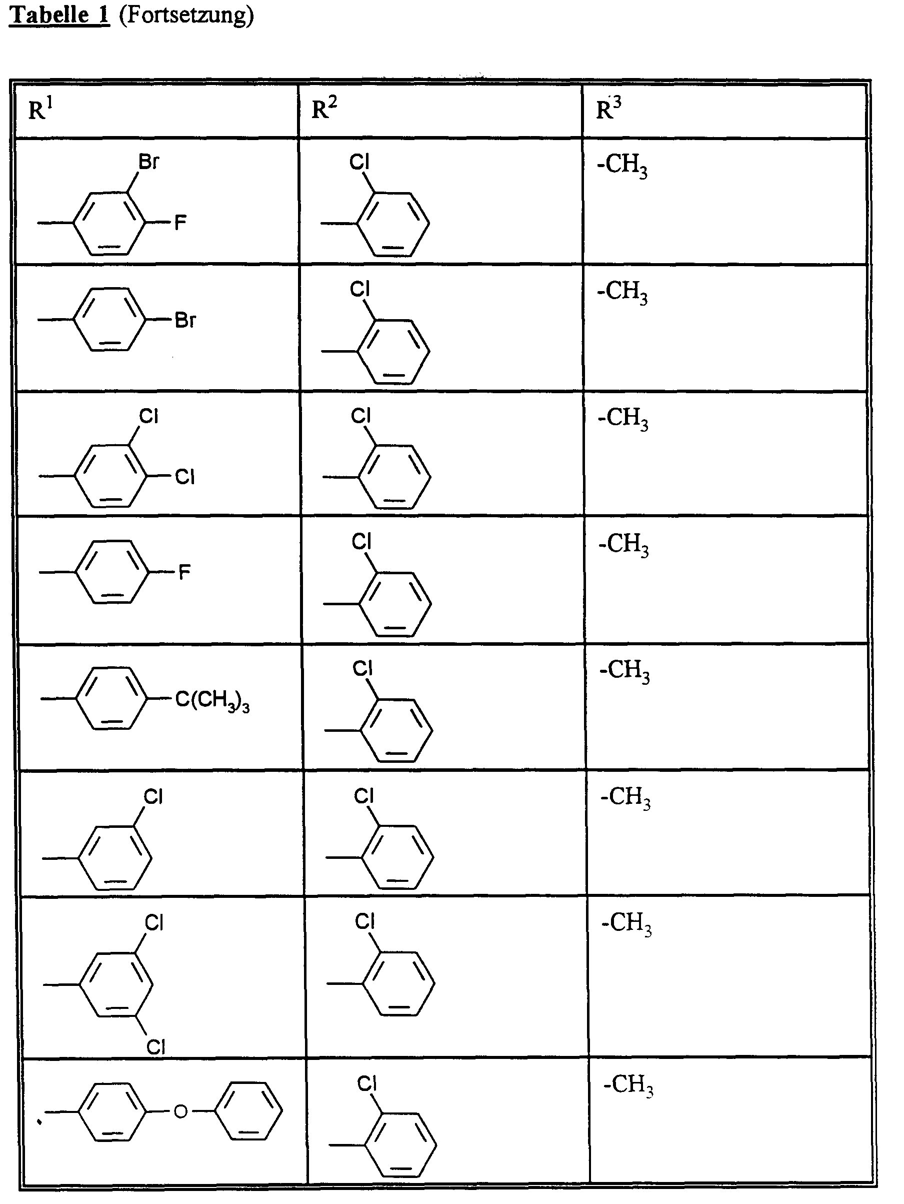

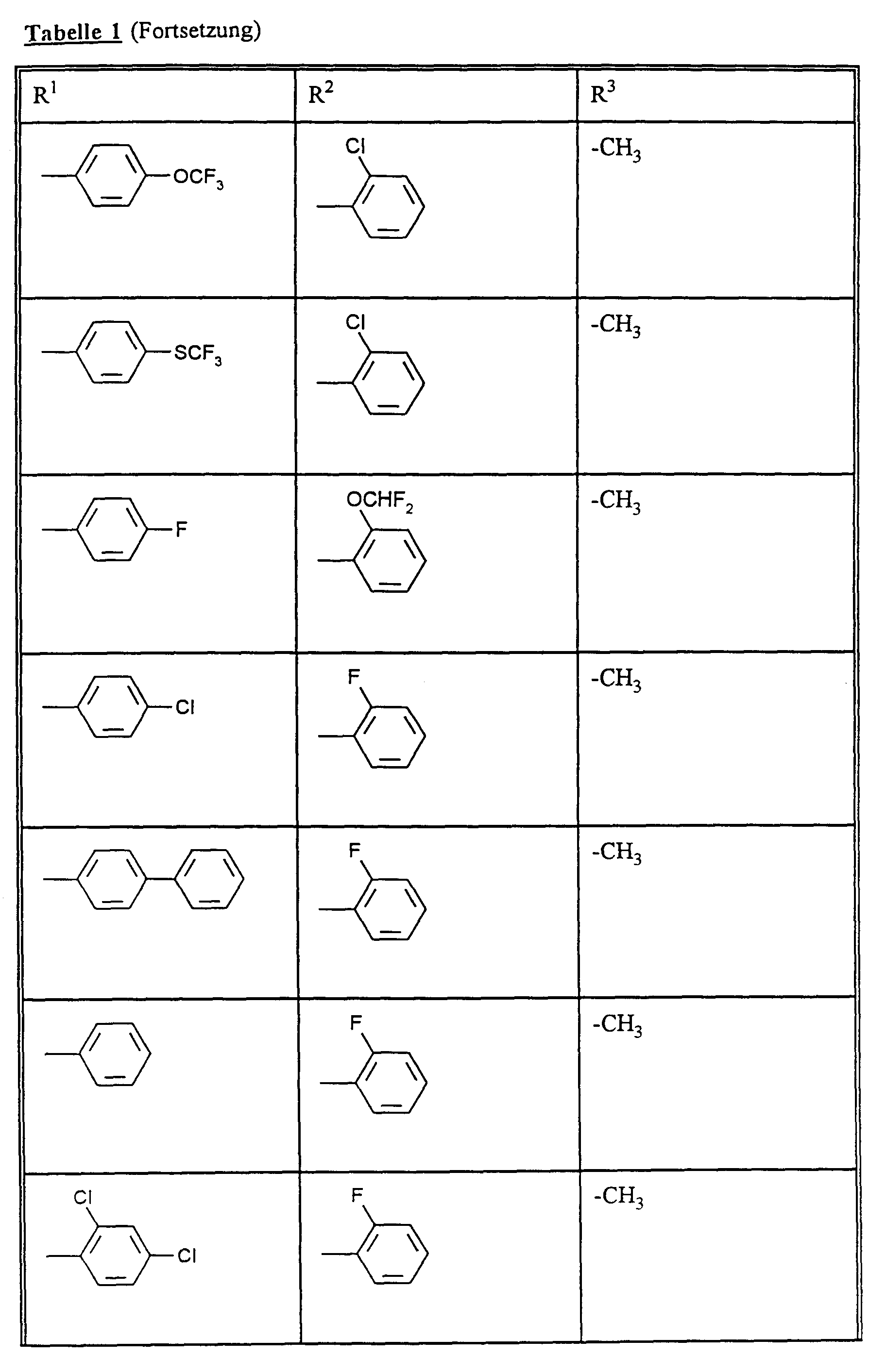

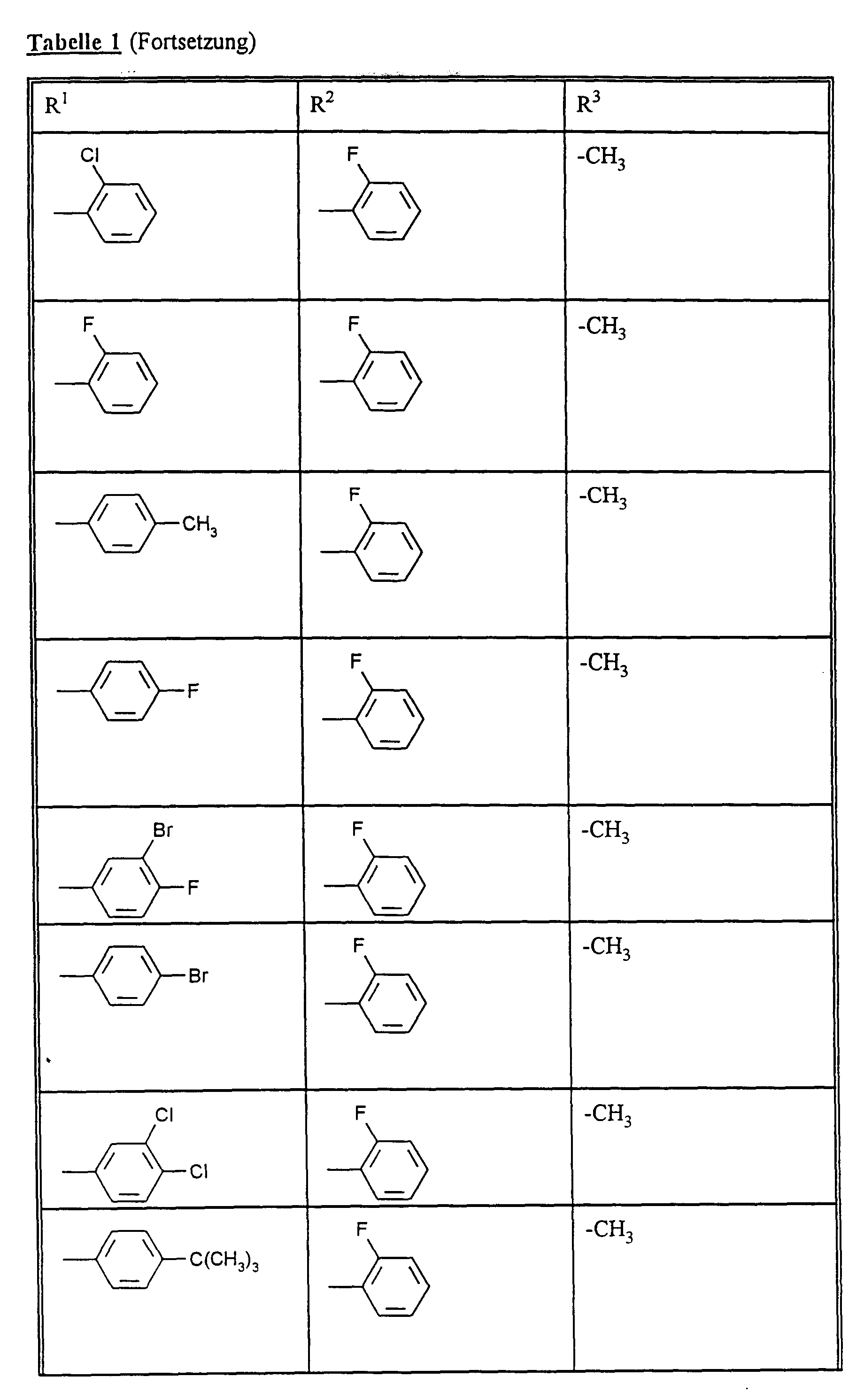

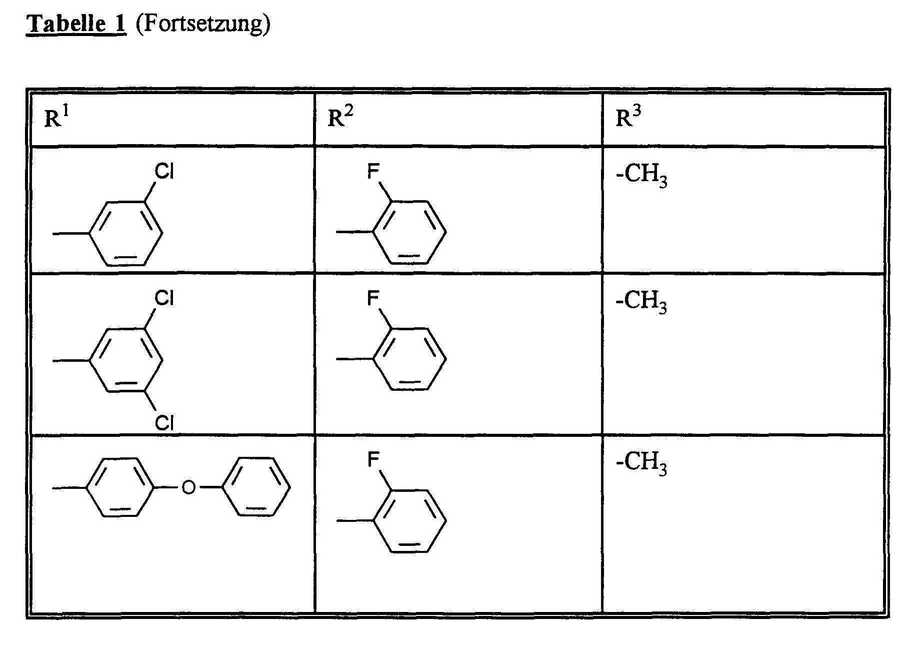

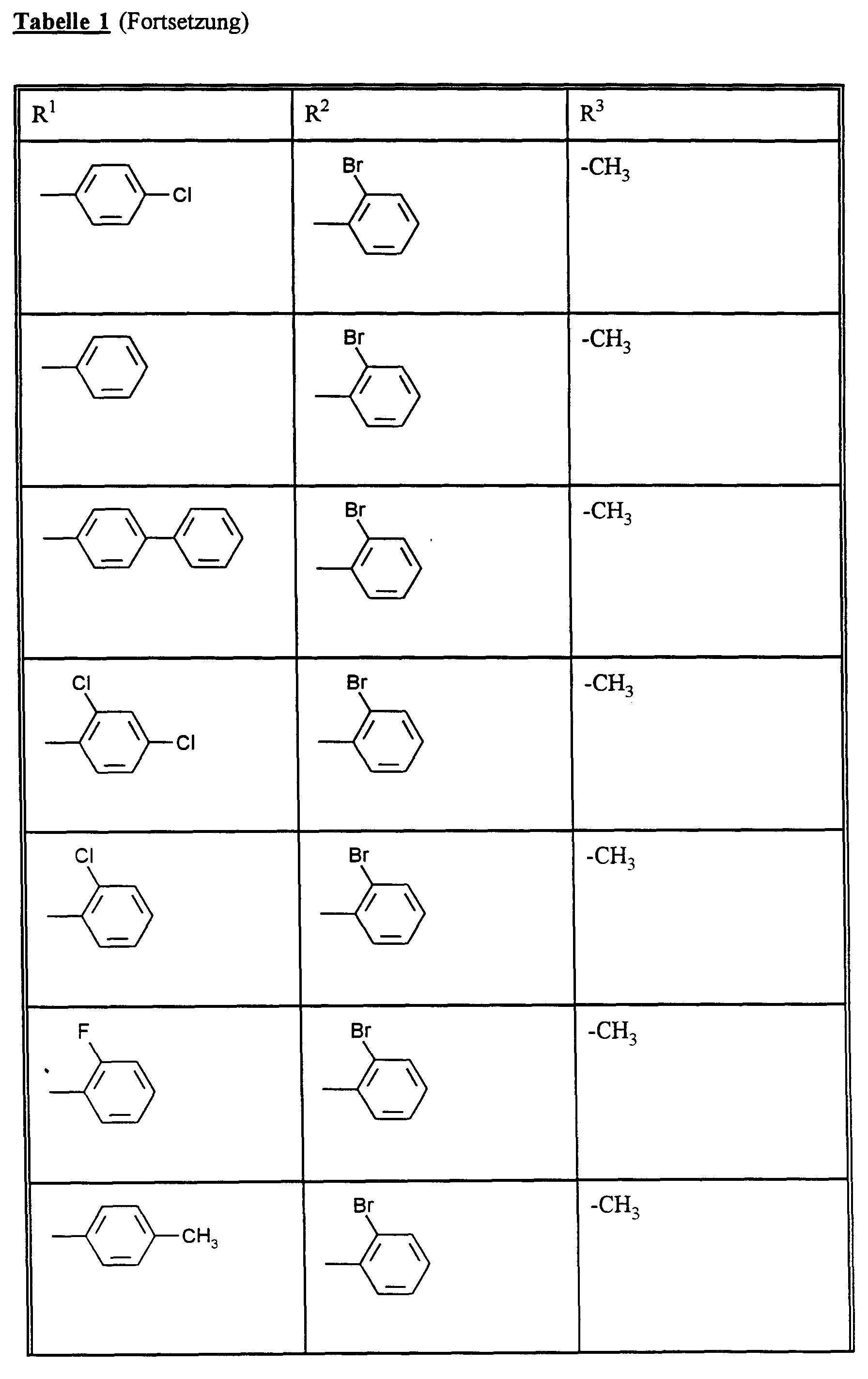

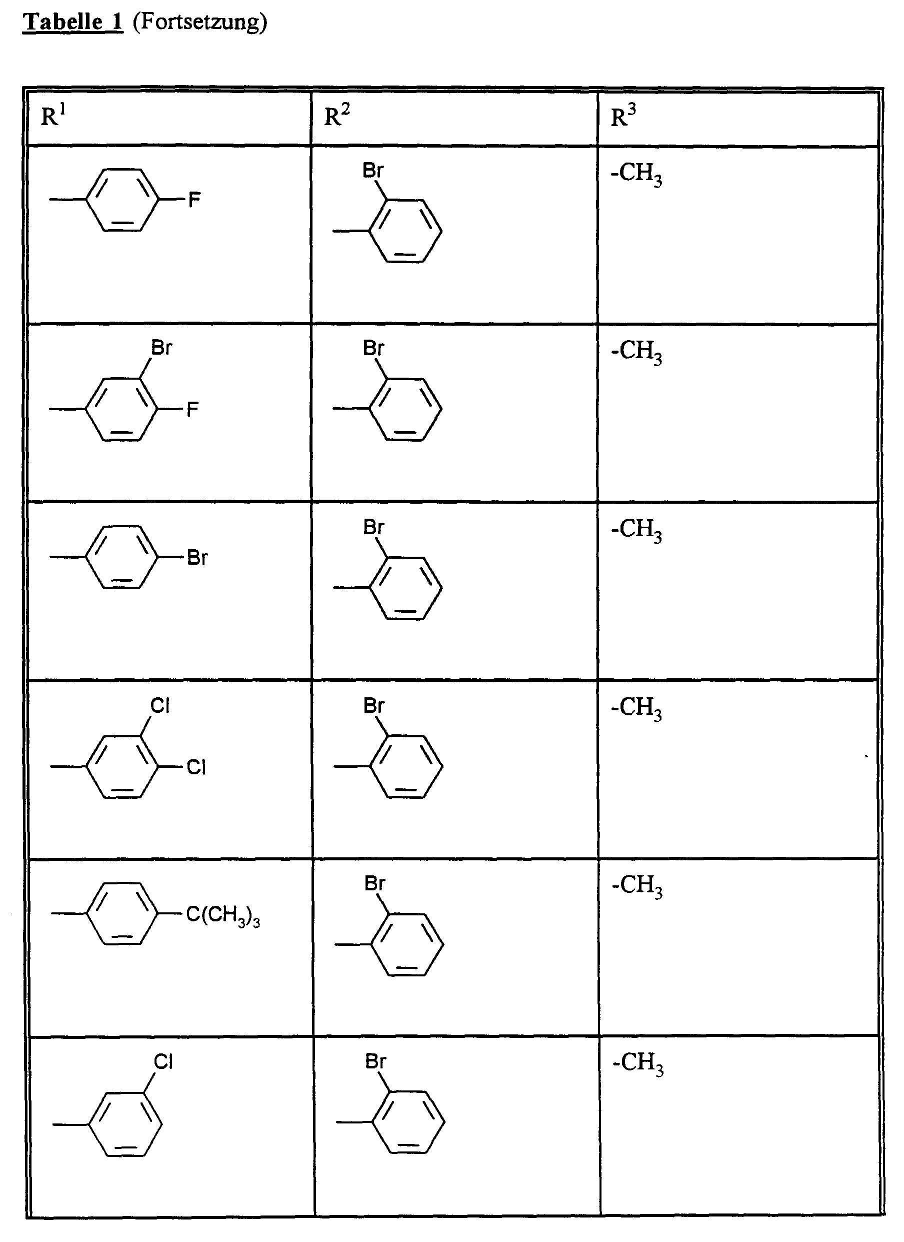

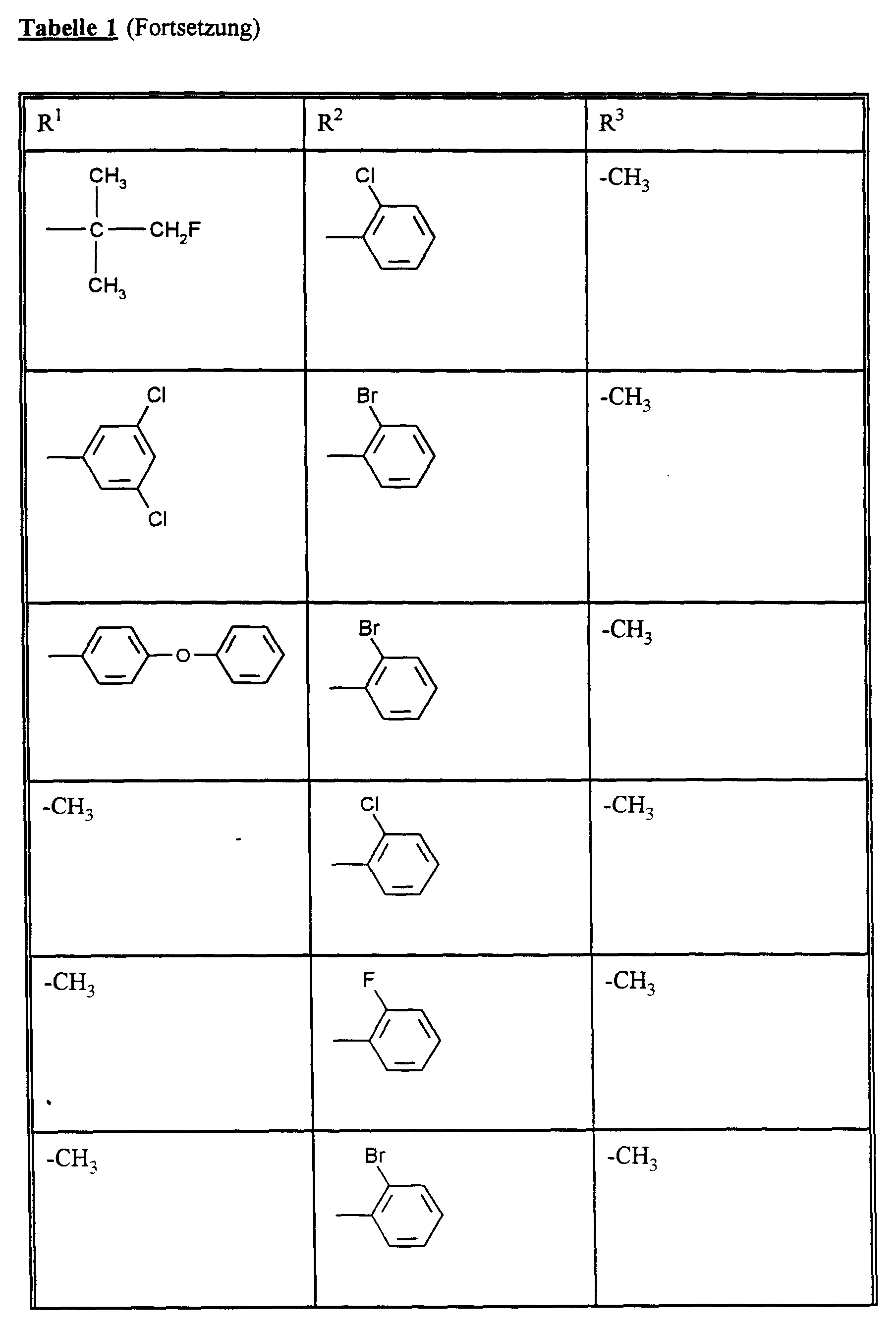

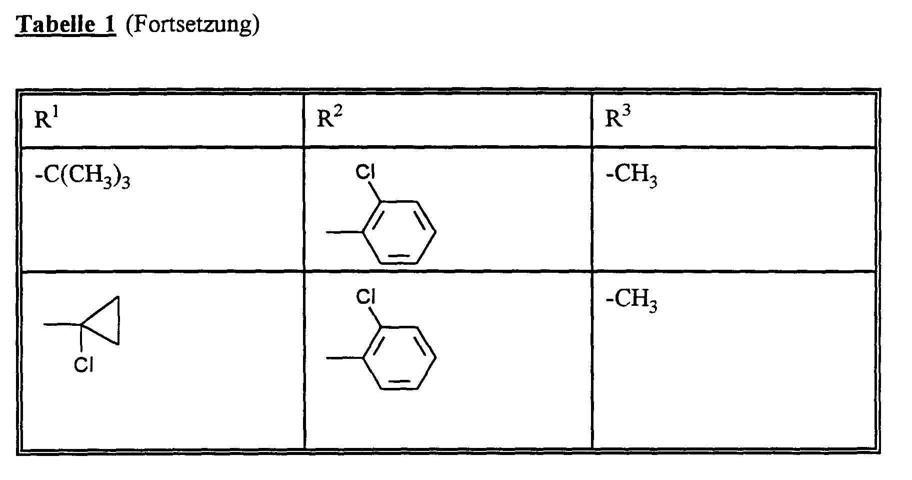

Verwendet man 3-(2-Chlor-phenyl)-2-(4-fluor-phenyl)-2-(1,2,4-triazol-1-yl-methyl)-oxiran (Z-Form) als Ausgangsstoff, n-Butyl-lithium als starke Base und Schwefel-Pulver als Reaktionskomponente, so kann der Verlauf der ersten Stufe des erfindungsgemäßen Verfahrens durch das folgende Formelschema veranschaulicht werden:

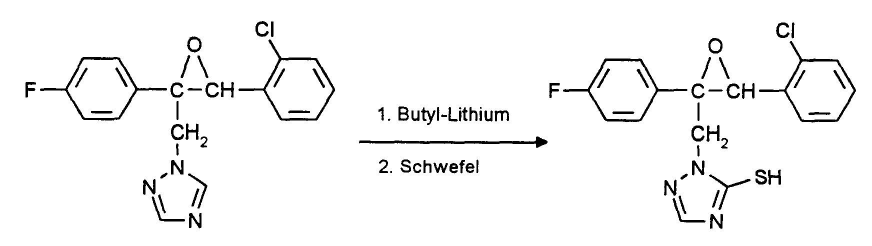

Verwendet man 3-(2-Chlor-phenyl)-2-(4-fluor-phenyl)-2-(5-mercapto-1,2,4-triazol-1-yl-methyl)-oxiran (Z-Form) als Ausgangsstoff und Methyliodid als Reaktionskomponente, so kann der Verlauf der zweiten Stufe des erfindungsgemäßen Verfahrens durch das folgende Formelschema veranschaulicht werden:

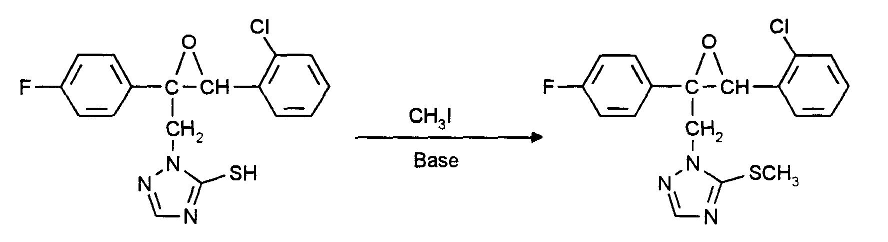

Die bei der Durchführung des erfindungsgemäßen Verfahrens als Ausgangsstoffe benötigten Oxiran-Derivate sind durch die Formel (II) allgemein definiert. In dieser Formel haben R¹ und R² vorzugsweise diejenigen Bedeutungen, die bereits im Zusammenhang mit der Beschreibung der erfindungsgemäßen Stoffe der Formel (I) vorzugsweise für diese Reste genannt wurden.

Die Oxiran-Derivate der Formel (II) sind bekannt oder lassen sich nach bekannten Methoden herstellen (vgl. EP-A 0 094 564 und EP-A 0 196 038).

Als Basen kommen bei der Durchführung der ersten Stufe des erfindungsgemäßen Verfahrens alle für derartige Reaktionen üblichen, starken Alkalimetall-Basen in Betracht. Vorzugsweise verwendbar sind n-Butyl-lithium, Lithium-diisopropylamid, Natriumhydrid, Natriumamid und auch Kalium-tert.-butylat im Gemisch mit Tetramethylethylen-diamin (= TMEDA).

Bei der Durchführung der ersten Stufe des erfindungsgemäßen Verfahrens kommen alle für derartige Umsetzungen üblichen inerten organischen Solventien als Verdünnungsmittel in Betracht. Vorzugsweise verwendbar sind Ether, wie Tetrahydrofuran, Dioxan, Diethylether und 1,2-Dimethoxyethan, ferner flüssiger Ammoniak oder auch stark polare Solventien, wie Dimethylsulfoxid.

Schwefel wird vorzugsweise in Form von Pulver eingesetzt. Zur Hydrolyse verwendet man bei der Durchführung der ersten Stufe des erfindungsgemäßen Verfahrens Wasser, gegebenenfalls in Gegenwart einer Säure. In Frage kommen hierbei alle für derartige Umsetzungen üblichen anorganischen oder organischen Säuren. Vorzugsweise verwendbar sind Essigsäure, verdünnte Schwefelsäure und verdünnte Salzsäure. Es ist jedoch auch möglich, die Hydrolyse mit wäßriger Ammoniumchlorid-Lösung durchzuführen.

Die Reaktionstemperaturen können bei der Durchführung der ersten Stufe des erfindungsgemäßen Verfahrens innerhalb eines bestimmten Bereiches variiert werden. Im allgemeinen arbeitet man bei Temperaturen zwischen -70°C und +20°C, vorzugsweise zwischen -70°C und 0°C.

Bei der Durchführung aller Schritte des erfindungsgemäßen Verfahrens arbeitet man im allgemeinen unter Normaldruck.

Bei der Durchführung der ersten Stufe des erfindungsgemäßen Verfahrens setzt man auf 1 Mol an Oxiran-Derivat der Formel (II) im allgemeinen 1 bis 3 Äquivalente, vorzugsweise 1,0 bis 2,5 Äquivalente, an starker Base und anschließend eine äquivalente Menge oder auch einen Überschuß an Schwefel ein. Die Umsetzung kann unter Schutzgas-atmosphäre, z.B. unter Stickstoff oder Argon. vorgenommen werden. Die Aufarbeitung erfolgt nach üblichen Methoden. Im allgemeinen geht man so vor, daß man das Reaktionsgemisch mit einem in Wasser wenig löslichen organischen Solvens extrahiert, die vereinigten organischen Phasen trocknet und einengt und den verbleibenden Rückstand gegebenenfalls durch Umkristallisation und/oder Chromatographie reinigt.

Die bei der Durchführung der zweiten Stufe des erfindungsgemäßen Verfahrens als Ausgangssubstanzen benötigten Verbindungen der Formel (Ia) sind erfindungsgemäße Stoffe.

Die bei der Durchführung des erfindungsgemäßen Verfahrens in der zweiten Stufe als Reaktionskomponenten benötigten Halogen-Verbindungen sind durch die Formel (III) allgemein definiert.
- R⁴: steht vorzugsweise für Methyl oder Ethyl.
- Hal: steht auch vorzugsweise für Chlor, Brom oder Iod.

Die Halogen-Verbindungen der Formel (III) sind bekannt.

Als Säurebindemittel kommen bei der Durchführung der zweiten Stufe des erfindungsgemäßen Verfahrens alle üblichen anorganischen oder organischen Basen in Frage. Vorzugsweise verwendbar sind Erdalkali- oder Alkalimetallhydroxide wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der zweiten Stufe des erfindungsgemäßen Verfahrens alle für derartige Umsetzungen üblichen, inerten organischen Solventien in Betracht. Vorzugsweise verwendbar sind Ether, wie Diethylether, Methyl-tert.-butyl-ether, Ethylenglykol-dimethylether, Tetrahydrofuran und Dioxan, ferner Nitrile, wie Acetonitril, und außerdem stark polare Solventien, wie Dimethylsulfoxid oder Dimethylformamid.

Die Reaktionstemperaturen können bei der Durchführung der zweiten Stufe des erfindungsgemäßen Verfahrens innerhalb eines größeren Bereiches variiert werden. Im allgemeinen arbeitet man bei Temperaturen zwischen 0°C und 120°C. vorzugsweise zwischen 20°C und 100°C.

Bei der Durchführung der zweiten Stufe des erfindungsgemäßen Verfahrens setzt man auf 1 Mol an Triazolyl-methyl-oxiran der Formel (Ia) im allgemeinen 1 bis 2 Mol an Halogen-Verbindung der Formel (III) sowie eine äquivalente Menge oder auch einen Überschuß an Säurebindemittel ein. Die Aufarbeitung erfolgt nach üblichen Methoden. Im allgemeinen geht man so vor, daß man das Reaktionsgemisch mit wäßriger Base und einem mit Wasser wenig mischbaren organischen Lösungsmittel versetzt, die organische Phase abtrennt, trocknet und einengt. Das erhaltene Produkt kann gegebenenfalls nach üblichen Methoden, z.B. durch Umkristallisation, von noch vorhandenen Verunreinigungen befreit werden.

Die nach dem erfindungsgemäßen Verfahren erhältlichen Triazolylmethyl-oxirane der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren isolieren und gegebenenfalls durch Umkristallisation reinigen.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Pseudocercosporella, Erysiphe- und Fusarium-Arten. Außerdem lassen sich die erfindungsgemäßen Stoffe sehr gut gegen Venturia und Sphaerotheca einsetzen. Sie besitzen darüber hinaus auch eine sehr gute in-vitro Wirkung.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden. wie Lösungen, Emulsionen, Suspensionen, Pulver, Schäume, Pasten, Granulate. Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel wie Alkohole als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe. wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Pflanzenschutz im allgemeinen zwischen 0,1 und 95 Gewichtsprozent Wirkstoff, vorzugsweise zwischen 0,5 und 90 %.

Die erfindungsgemäßen Wirkstoffe können bei Verwendung im Pflanzenschutz in den Formulierungen in Mischung mit bekannten Fungiziden, Bakteriziden, Akariziden, Nematiziden oder Insektiziden eingesetzt werden, um so z.B. das Wirkungsspektrum zu verbreitern oder Resistenzentwicklungen vorzubeugen. In manchen Fällen kann auch Synergismus auftreten.

Für die Mischungen kommen beispielsweise folgende Stoffe in Frage.

**Fungizide:**
2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoximino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil, Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin. Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin. Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron. Diazinon, Dichlofenthion, Dichlorvos, Dicliphos, Dicrotophos, Diethion. Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen, Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen errmittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung der erfindungsgemäßen Stoffe geht aus den folgenden Beispielen hervor.

### Herstellungsbeispiele

### Beispiel 1

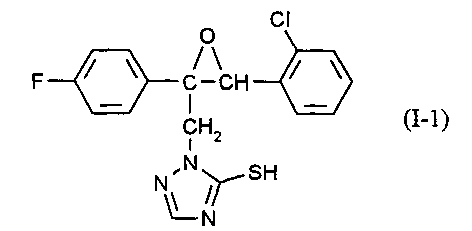

Ein Gemisch aus 1,3 g (4 mmol) 3-(2-Chlor-phenyl)-2-(4-fluor-phenyl)-2-(1,2,4-triazol-1-yl-methyl)-oxiran (Z-Form) und 25 ml absolutem Tetrahydrofuran wird bei -70°C mit 2,0 ml (5 mmol) n-Butyl-lithium in Hexan versetzt und 1 Stunde bei -70°C gerührt. Danach wird das Reaktionsgemisch mit 0,16 g (5 mmol) Schwefel-Pulver versetzt und 4 Stunden bei -70°C gerührt. Anschließend werden bei -70°C gleichzeitig 1 ml Methanol und 1 ml Essigsäure unter Rühren zugetropft. Man verdünnt das entstehende Gemisch mit Dichlormethan und schüttelt mehrfach mit gesättigter, wäßriger Ammoniumchlorid-Lösung aus. Die organische Phase wird über Natriumsulfat getrocknet und dann unter vermindertem Druck eingeengt. Das anfallende Rohprodukt (1,9 g), das gemäß Gaschromatogramm neben 20,7 % an Ausgangssubstanz 51,0 % an dem gewünschten Produkt enthält, wird aus Toluol umkristallisiert. Man erhält auf diese Weise 0,8 g (55 % der Theorie) an 3-(2-Chlorphenyl)-2-(4-fluor-phenyl)-2-(5-mercapto-1,2,4-triazol-1-yl-methyl)-oxiran (Z-Form) als Festsubstanz vom Schmelzpunkt 179 bis 180°C.
¹H-NMR-Spektrum (200 MHz, CDCl₃, TMS):
δ = 3,7 (d, J=15 Hz, 1H); 4,1 (s, 1H); 5,15 (d, J=15 Hz, 1H); 6,95-7,6 (m, 8H); 7,65 (s, 1H); 11,0 (SH) ppm.
GC/MS (ci): 362 (M+H⁺)

### Verwendungsbeispiele

In den folgenden Verwendungsbeispielen wurde die nachstehend aufgeführte Verbindung als Vergleichssubstanz eingesetzt:

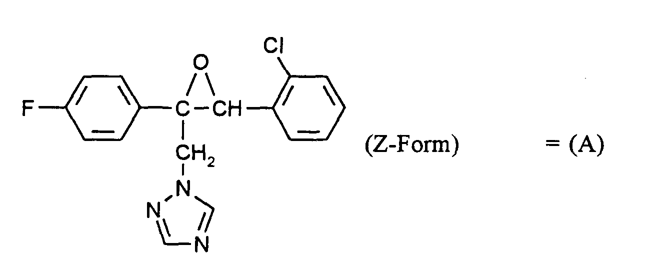

Bekannt aus EP-A 0 196 038.

### Beispiel A

Pseudocercosporella herpotrichoides-Test (Weizen) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

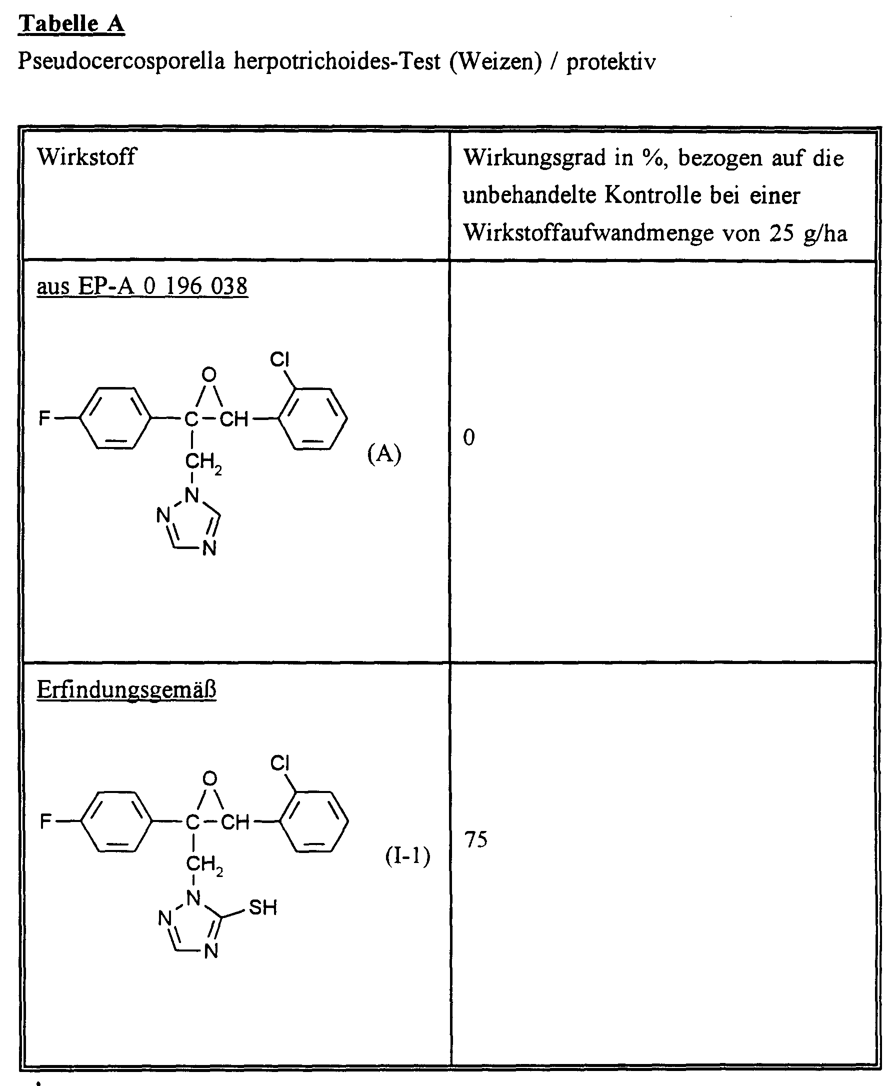

### Beispiel B

Fusarium culmorum-Test (Weizen) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit einer Konidiensuspension von Fusarium culmorum besprüht.

Die Pflanzen werden in einem Gewächshaus unter lichtdurchlässigen Inkubationshauben bei einer Temperatur von ca. 20°C und einer relativen Luftfeuchtigkeit von ca. 100 % aufgestellt.

4 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

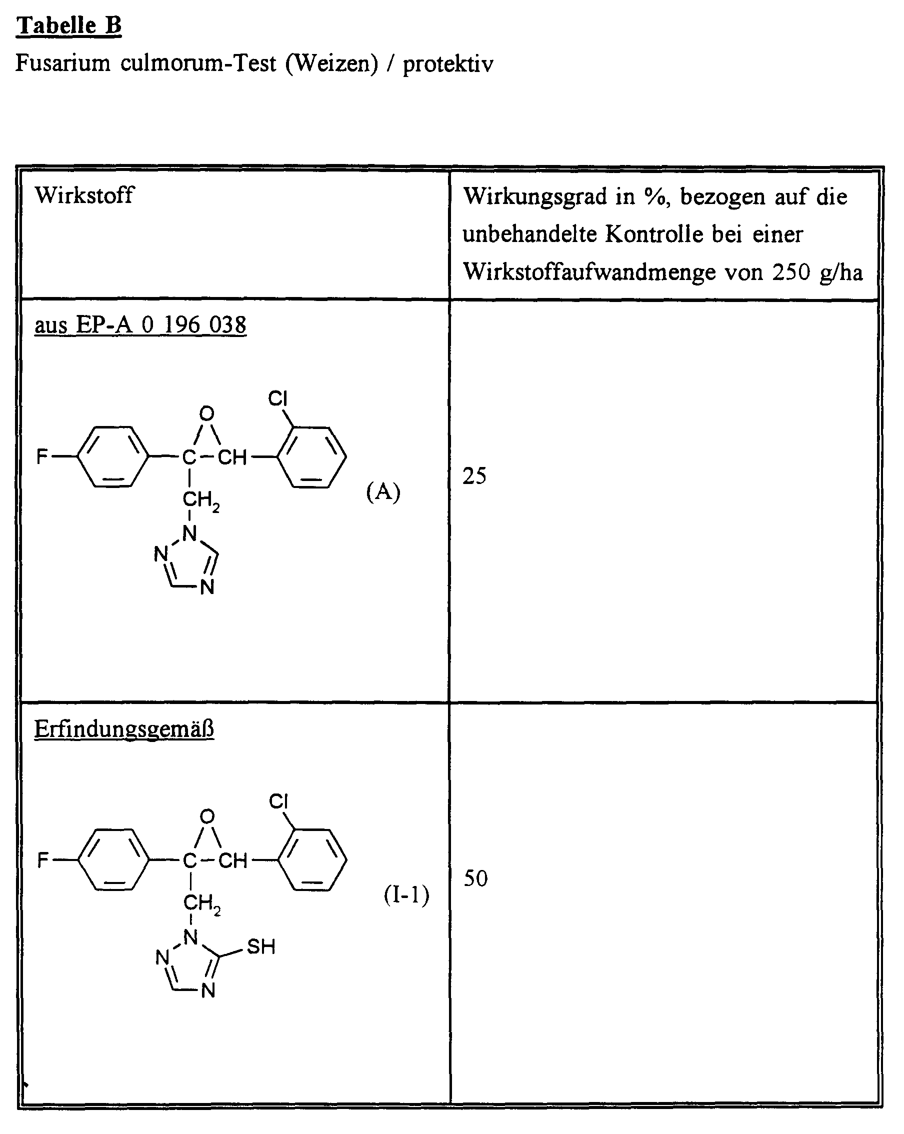

### Beispiel C

Sphaerotheca-Test (Gurke) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

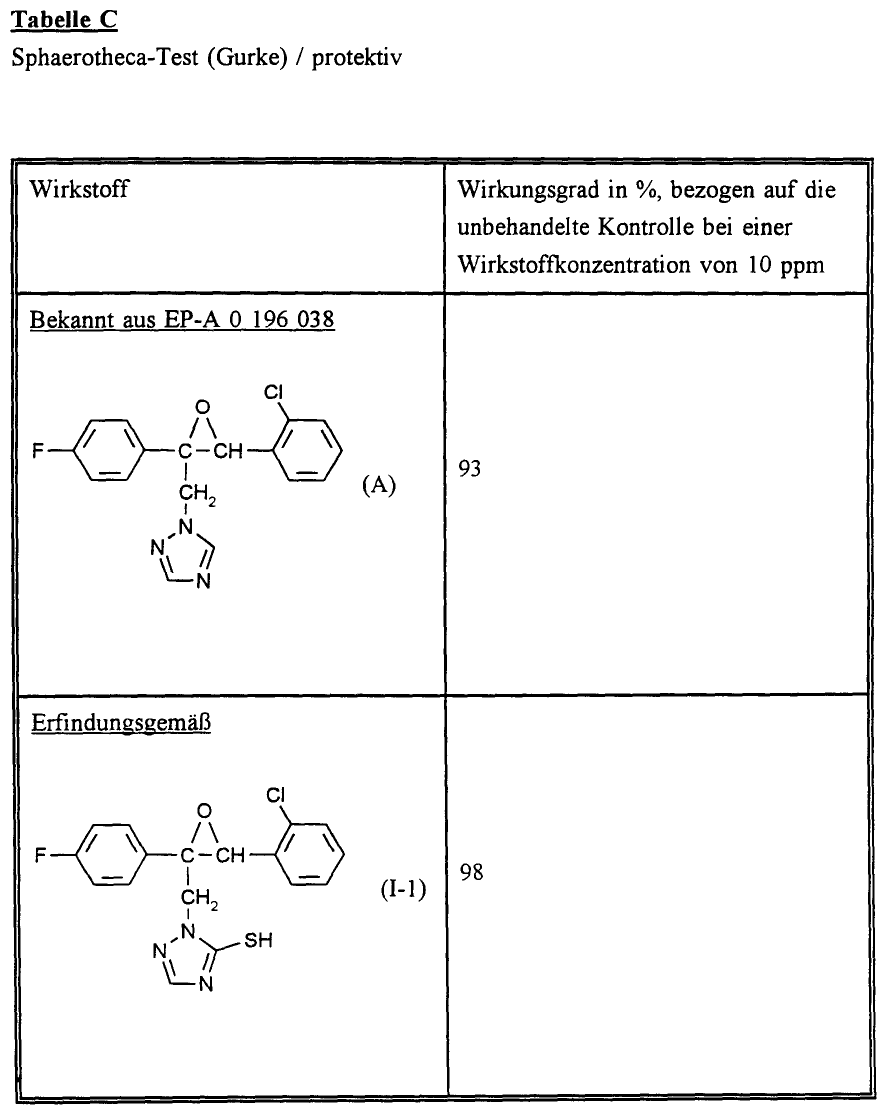

### Beispiel D

PodosphaeraTest (Apfel) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23°C und einer relativen Luftfeuchtigkeit von ca. 70 % aufgestellt.

10 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

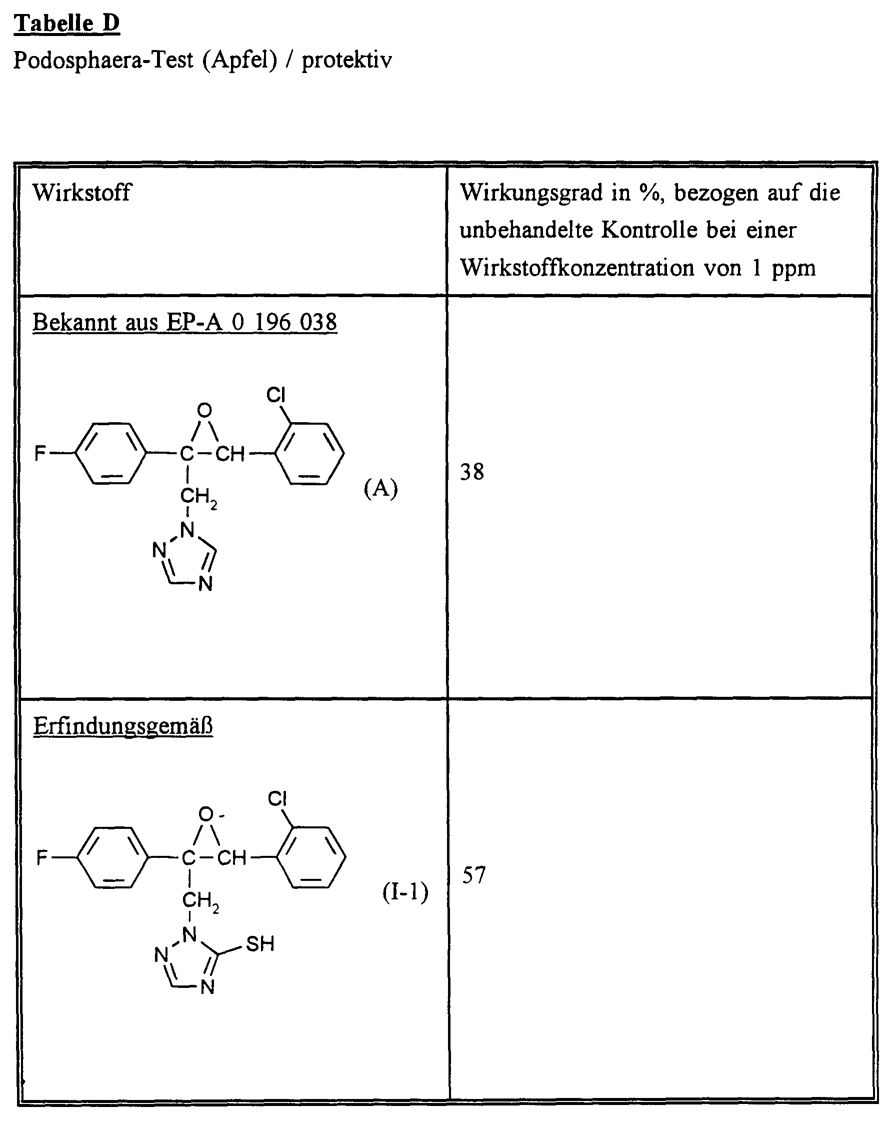

## Patentansprüche

1. Triazolylmethyl-oxirane der Formel 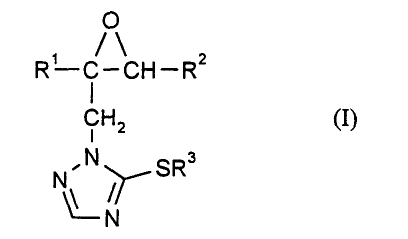 in welcher
R¹ für Alkyl mit 1 bis 4 Kohlenstoffatomen, Halogenalkyl mit 1 bis 4 Kohlenstoffatomen und 1 bis 5 Halogenatomen, gegebenenfalls durch Halogen substituiertes Cycloalkyl mit 3 bis 7 Kohlenstoffatomen, Naphthyl oder gegebenenfalls einfach bis dreifach, gleichartig oder verschieden durch Halogen, Nitro, Phenyl, Phenoxy, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen und/oder Halogenalkylthio mit 1 bis 4 Kohlenstoffatomen und 1 bis 5 Halogenatomen substituiertes Phenyl steht,
R² für gegebenenfalls einfach bis dreifach, gleichartig oder verschieden durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen und/oder Halogenalkylthio mit 1 bis 4 Kohlenstoffatomen und 1 bis 5 Halogenatomen substituiertes Phenyl steht und
R³ für Wasserstoff oder Alkyl mit 1 bis 4 Kohlenstoffatomen steht,
sowie deren Säureadditions-Salze und Metallsalz-Komplexe.

2. Triazolylmethyl-oxirane der Formel (I) gemäß Anspruch 1, in denen
R¹ für Methyl, Ethyl, n-Propyl, Isopropyl, n-Butyl, sek.-Butyl, iso-Butyl, tert.-Butyl, Fluor-tert.-butyl, Difluor-tert.-butyl, gegebenenfalls einfach bis dreifach, gleichartig oder verschieden durch Fluor, Chlor und/oder Brom substituiertes Cycloalkyl mit 3 bis 6 Kohlenstoffatomen, für Naphthyl oder für Phenyl steht, das einfach bis dreifach, gleichartig oder verschieden substituiert sein kann durch Fluor, Chlor, Brom, Nitro, Phenyl, Phenoxy, Methyl, Ethyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio,
R² für Phenyl steht, das einfach bis dreifach, gleichartig oder verschieden substituiert sein kann durch Fluor, Chlor, Brom, Methyl, Ethyl, Isopropyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio, und
R³ für Wasserstoff, Methyl oder Ethyl steht.

3. Verfahren zur Herstellung von Triazolylmethyl-oxiranen der Formel (I) gemäß Anspruch 1 sowie von deren Säureadditions-Salzen und Metallsalz-Komplexen, dadurch gekennzeichnet, daß man Oxiran-Derivate der Formel 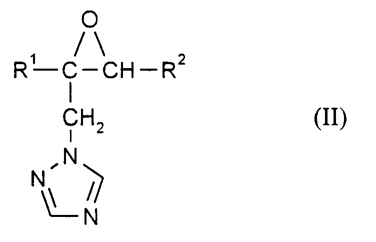 in welcher
R¹ und R² die oben angegebenen Bedeutungen haben,
nacheinander mit starken Basen und Schwefel in Gegenwart eines Verdünnungsmittels umsetzt und dann mit Wasser, gegebenenfalls in Gegenwart einer Säure hydrolysiert und gegebenenfalls die dabei entstehenden Verbindungen der Formel 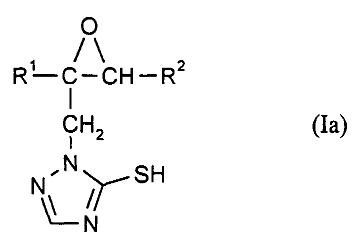 in welcher
R¹ und R² die oben angegebenen Bedeutungen haben,
mit Halogen-Verbindungen der Formel
R⁴-Hal (IV)
in welcher
R⁴ für Alkyl mit 1 bis 4 Kohlenstoffatomen steht und
Hal für Chlor, Brom oder Iod steht,
in Gegenwart eines Säurebindemittels und in Gegenwart eines Verdünnungsmittels umsetzt,
und gegebenenfalls anschließend an die so erhaltenen Verbindungen der Formel (I) eine Säure oder ein Metallsalz addiert.

4. Mikrobizide Mittel, gekennzeichnet durch einen Gehalt an mindestens einem Triazolylmethyl-oxiran der Formel (I) gemäß Anspruch 1 bzw. an einem Säureadditionssalz oder Metallsalz-Komplex eines Triazolylmethyloxirans der Formel (I).

5. Verwendung von Triazolylmethyl-oxiranen der Formel (I) gemäß Anspruch 1 bzw. von deren Säureadditionssalzen oder Metallsalz-Komplexen als Mikrobizide im Pflanzenschutz und im Materialschutz.

6. Verfahren zur Bekämpfung von unerwünschten Mikroorganismen im Pflanzenschutz und im Materialschutz, dadurch gekennzeichnet, daß man Triazolylmethyl-oxirane der Formel (I) gemäß Anspruch 1 bzw. deren Säureadditionssalze oder Metallsalz-Komplexe auf die Mikroorganismen und/oder deren Lebensraum ausbringt.

7. Verfahren zur Herstellung von mikrobiziden Mitteln, dadurch gekennzeichnet, daß man Triazolylmethyl-oxirane der Formel (I) gemäß Anspruch 1 bzw. deren Säureadditionssalze oder Metallsalz-Komplexe mit Streckmitteln und/oder oberflächenaktiven Stoffen vermischt.

## Claims

1. Triazolylmethyl-oxiranes of the formula 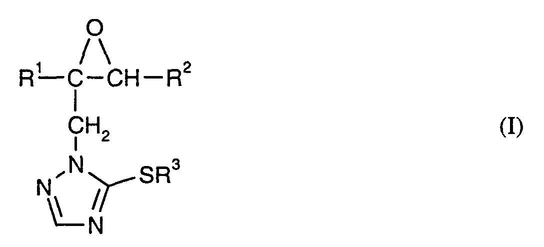 in which
R¹ represents alkyl having 1 to 4 carbon atoms, halogenoalkyl having 1 to 4 carbon atoms and 1 to 5 halogen atoms, cycloalkyl having 3 to 7 carbon atoms which is optionally substituted by halogen, or represents naphthyl or represents phenyl which is optionally substituted from one to three times by identical or different substituents consisting of halogen, nitro, phenyl, phenoxy, alkyl having 1 to 4 carbon atoms, alkoxy having 1 to 4 carbon atoms, halogenoalkyl having 1 to 4 carbon atoms and 1 to 5 halogen atoms, halogenoalkoxy having 1 to 4 carbon atoms and 1 to 5 halogen atoms and/or halogenoalkylthio having 1 to 4 carbon atoms and 1 to 5 halogen atoms,
R² represents phenyl which is optionally substituted from one to three times by identical or different substituents consisting of halogen, alkyl having 1 to 4 carbon atoms, alkoxy having 1 to 4 carbon atoms, halogenoalkyl having 1 to 4 carbon atoms and 1 to 5 halogen atoms, halogenoalkoxy having 1 to 4 carbon atoms and 1 to 5 halogen atoms and/or halogenoalkylthio having 1 to 4 carbon atoms and 1 to 5 halogen atoms, and
R³ represents hydrogen or alkyl having 1 to 4 carbon atoms,
and their acid addition salts and metal salt complexes.

2. Triazolylmethyl-oxiranes of the formula (I) according to Claim 1, in which
R¹ represents methyl, ethyl, n-propyl, isopropyl, n-butyl, sec-butyl, isobutyl, tert-butyl, fluoro-tert-butyl, difluoro-tert-butyl, cycloalkyl having 3 to 6 carbon atoms which is optionally substituted from one to three times by identical or different substituents consisting of fluorine, chlorine and/or bromine, or represents naphthyl or represents phenyl which can be substituted from one to three times by identical or different substituents consisting of fluorine, chlorine, bromine, nitro, phenyl, phenoxy, methyl, ethyl, tert-butyl, methoxy, ethoxy, trifluoromethyl, trichloromethyl, difluoromethyl, difluorochloromethyl, trifluoromethoxy, difluoromethoxy and/or trifluoromethylthio,
R² represents phenyl which can be substituted from one to three times by identical or different substituents consisting of fluorine, chlorine, bromine, methyl, ethyl, isopropyl, tert-butyl, methoxy, ethoxy, trifluoromethyl, trichloromethyl, difluoromethyl, difluorochloromethyl, trifluoromethoxy, difluoromethoxy and/or trifluoromethylthio, and
R³ represents hydrogen, methyl or ethyl.

3. Process for the preparation of triazolylmethyl-oxiranes of the formula (I) according to Claim 1 and of their acid addition salts and metal salt complexes, characterized in that oxirane derivatives of the formula 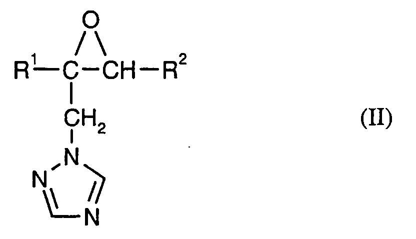 in which
R¹ and R² have the meanings given above,
are reacted in succession with strong bases and sulphur in the presence of a diluent and the reaction product is then hydrolysed with water, optionally in the presence of an acid, and the resulting compounds of the formula 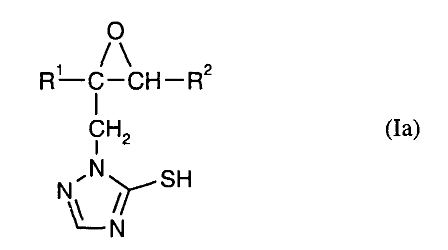 in which
R¹ and R² have the meanings given above
are optionally reacted with halogen compounds of the formula
R⁴-Hal (IV)
in which
R⁴ represents alkyl having 1 to 4 carbon atoms and
Hal represents chlorine, bromine or iodine,
in the presence of an acid-binding agent and in the presence of a diluent,
and then an acid or a metal salt is optionally added onto the compounds of the formula (I) thus obtained.

4. Microbicidal compositions, characterized by the presence of at least one triazolylmethyl-oxirane of the formula (I) according to Claim 1 and/or of an acid addition salt or metal salt complex of a triazolylmethyl-oxirane of the formula (I).

5. Use of triazolylmethyl-oxiranes of the formula (I) according to Claim 1 and/or of their acid addition salts or metal salt complexes as microbicides in plant protection and in the protection of materials.

6. Method of combating unwanted microorganisms in plant protection and in the protection of materials, characterized in that triazolylmethyl-oxiranes of the formula (I) according to Claim 1 and/or their acid addition salts or metal salt complexes are applied to the microorganisms and/or their habitat.

7. Process for the production of microbicidal compositions, characterized in that triazolylmethyl-oxiranes of the formula (I) according to Claim 1 and/or their acid addition salts or metal salt complexes are mixed with extenders and/or surface-active substances.

## Revendications

1. Triazolylméthyloxirannes de formule 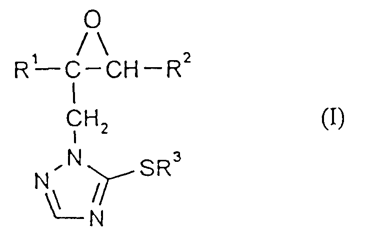 dans laquelle
R¹ est un groupe alkyle ayant 1 à 4 atomes de carbone, un groupe halogénalkyle ayant 1 à 4 atomes de carbone et 1 à 5 atomes d'halogènes, un groupe cycloalkyle de 3 à 7 atomes de carbone éventuellement substitué par un halogène, un groupe naphtyle ou un groupe phényle éventuellement substitué une à trois fois identiques ou différentes par un reste halogéno, nitro, phényle, phénoxy, alkyle ayant 1 à 4 atomes de carbone, alkoxy ayant 1 à 4 atomes de carbone, halogénalkyle ayant 1 à 4 atomes de carbone et 1 à 5 atomes d'halogènes, halogénalkoxy ayant 1 à 4 atomes de carbone et 1 à 5 atomes d'halogènes et/ou halogénalkylthio ayant 1 à 4 atomes de carbone et 1 à 5 atomes d'halogènes,
R² est un groupe phényle éventuellement substitué une à trois fois identiques ou différentes par un reste halogéno, alkyle ayant 1 à 4 atomes de carbone, alkoxy ayant 1 à 4 atomes de carbone, halogénalkyle ayant 1 à 4 atomes de carbone et 1 à 5 atomes d'halogènes, halogénalkoxy ayant 1 à 4 atomes de carbone et 1 à 5 atomes d'halogènes et/ou halogénalkylthio ayant 1 à 4 atomes de carbone et 1 à 5 atomes d'halogènes et
R³ est l'hydrogène ou un groupe alkyle ayant 1 à 4 atomes de carbone
ainsi que leurs sels d'addition d'acides et leurs complexes avec des sels métalliques.

2. Triazolylméthyloxirannes de formule (I) suivant la revendication 1, dans lesquels
R¹ est un groupe méthyle, éthyle, n-propyle, isopropyle, n-butyle, sec.-butyle, isobutyle, tertio-butyle, fluoro-tertio-butyle, difluoro-tertio-butyle, un groupe cycloalkyle de 3 à 6 atomes de carbone éventuellement substitué une à trois fois identiques ou différentes par du fluor, du chlore et/ou du brome, un groupe naphtyle ou un groupe phényle qui peut être substitué une à trois fois identiques ou différentes par du fluor, du chlore, du brome, un reste nitro, phényle, phénoxy, méthyle, éthyle, tertio-butyle, méthoxy, éthoxy, trifluorométhyle, trichlorométhyle, difluorométhyle, difluorochlorométhyle, trifluorométhoxy, difluorométhoxy et/ou trifluorométhylthio,
R² est un groupe phényle qui peut être substitué une à trois fois identiques ou différentes par du fluor, du chlore, du brome, un reste méthyle, éthyle, isopropyle, tertio-butyle, méthoxy, éthoxy, trifluorométhyle, trichlorométhyle, difluorométhyle, difluorochlorométhyle, trifluorométhoxy, difluorométhoxy et/ou trifluorométhylthio, et
R³ est l'hydrogène, le groupe méthyle ou le groupe éthyle.

3. Procédé de production de triazolylméthyloxirannes de formule (I) suivant la revendication 1, ainsi que de leurs sels d'addition d'acides et de leurs complexes avec des sels métalliques, caractérisé en ce qu'on fait réagir des dérivés d'oxiranne de formule 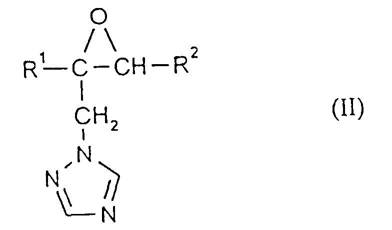 dans laquelle
R¹ et R² ont les définitions indiquées ci-dessus,
successivement avec des bases fortes et du soufre en présence d'un diluant, puis on effectue une hydrolyse avec de l'eau, le cas échéant en présence d'un acide et, le cas échéant, on fait réagir les composés ainsi produits de formule 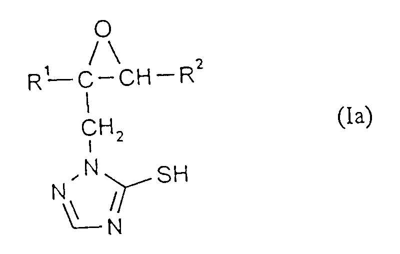 dans laquelle
R¹ et R² ont les définitions indiquées ci-dessus, avec des composés halogénés de formule
R⁴-Hal (IV)
dans laquelle
R⁴ est un groupe alkyle ayant 1 à 4 atomes de carbone et
Hal est le chlore, le brome ou l'iode,
en présence d'un accepteur d'acide et en présence d'un diluant,
puis, le cas échéant, on additionne sur les composés de formule (I) ainsi obtenus un acide ou un sel métallique.

4. Compositions microbicides, caractérisées par une teneur en au moins un triazolylméthyloxiranne de formule (I) suivant la revendication 1 ou en un sel d'addition d'acide ou en un complexe de sel métallique d'un triazolylméthyloxiranne de formule (I).

5. Utilisation de triazolylméthyloxirannes de formule (I) suivant la revendication 1 ou de leurs sels d'addition d'acides ou de leurs complexes de sels métalliques comme microbicides dans la protection des plantes et dans la protection des matériaux.

6. Procédé pour combattre des micro-organismes indésirables dans la protection des plantes et dans la protection des matériaux, caractérisé en ce qu'on épand des triazolylméthyloxirannes de formule (I) suivant la revendication 1 ou leurs sels d'addition d'acides ou leurs complexes de sels métalliques sur les micro-organismes et/ou sur leur milieu.

7. Procédé de préparation de compositions microbicides, caractérisé en ce qu'on mélange des triazolylméthyloxirannes de formule (I) suivant la revendication 1 ou leurs sels d'addition d'acides ou leurs complexes de sels métalliques avec des diluants et/ou des agents tensio-actifs.
